(19) Europäisches Patentamt
European Patent Office
Office européen des brevets

(11) Veröffentlichungsnummer: **0 056 617**
**B1**

(12) **EUROPÄISCHE PATENTSCHRIFT**

(45) Veröffentlichungstag der Patentschrift: **13.11.85**

(21) Anmeldenummer: **82100204.5**

(22) Anmeldetag: **13.01.82**

(51) Int. Cl.[4]: **C 07 D 295/02**, A 61 K 31/645 // C07C23/18, C07C35/37, C07C33/38, C07C57/38, C07C69/616, C07C69/618, C07C143/36, C07D295/18

# (54) Cycloheptenderivate, Verfahren und Zwischenprodukte für ihre Herstellung, sowie diese enthaltende Arzneimittel.

(30) Priorität: **16.01.81 CH 273/81**

(43) Veröffentlichungstag der Anmeldung: **28.07.82 Patentblatt 82/30**

(45) Bekanntmachung des Hinweises auf die Patenterteilung: **13.11.85 Patentblatt 85/46**

(84) Benannte Vertragsstaaten: **AT BE CH DE FR GB IT LI LU NL SE**

(56) Entgegenhaltungen:
**GB - A - 1 017 696**
**US - A - 3 409 640**
**US - A - 4 127 717**

**HELVETICA CHIMICA ACTA, Band 53, Nr. 5, 1970 BASEL (CH) P. DOSTERT et al.: "Nr. 105. Synthèse par réaction de Friedel-Crafts d'analogues partiellement saturés de l'amitriptyline" Seiten 882-897**
**HELVETICA CHIMICA ACTA, Band 53, Nr. 5, 1970 BASEL (CH) P. DOSTERT et al.: "Nr. 106. Synthèse d'analogues partiellement saturés de l'amitriptyline à partir d'ortho-tolunitriles" Seiten 897-904**
**J. Med. Chem., 14 (1971), 331-35**

(73) Patentinhaber: **F. HOFFMANN-LA ROCHE & CO. Aktiengesellschaft, CH-4002 Basel (CH)**

(72) Erfinder: **Aschwanden, Werner, Grellingerstrasse 4, CH-4107 Ettingen (CH)**
Erfinder: **Branca, Quirico, Dr., Lenzgasse 2, CH-4056 Basel (CH)**
Erfinder: **Kyburz, Emilio, Dr., Unterer Rebbergweg 127, CH-4153 Reinach (CH)**
Erfinder: **Pfister, Rudolf, Dr., Neubadstrasse 128, CH-4054 Basel (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## Beschreibung

Die Erfindung betrifft 1-[2-(4,5,10,11-Tetrahydro-1H-dibenzo[a,d]cyclohepten-5-yl)äthyl]pyrrolidin der Formel I

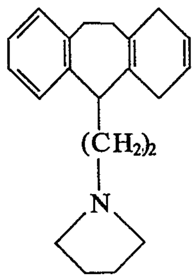

(I)

und pharmazeutisch annehmbare Säureadditionssalze davon.

Diese Verbindungen wurden bisher noch nicht beschrieben; sie besitzen wertvolle therapheutische Eigenschaften und können bei der Bekämpfung bzw. Verhütung von Krankheiten verwendet werden.

Gegenstand der Erfindung sind die Verbindung der Formel I und ihre pharmazeutisch annehmbaren Säureadditionssalze als solche und als pharmazeutische Wirkstoffe, Verfahren und Zwischenprodukte zu ihrer Herstellung, ferner Arzneimittel enthaltend die Verbindung der Formel I oder ein pharmazeutisch annehmbares Säureadditionssalz davon und ein Verfahren zur Herstellung dieser Arzneimittel.

Der in dieser Beschreibung verwendete Ausdruck »Abgangsgruppe« umfaßt Halogenatome, wie Chlor, Brom und Jod, Sulfonsäurereste, wie Methansulfonyloxy, p-Toluolsulfonyloxy, p-Brombenzolsulfonyloxy oder Benzolsulfonyloxy und andere äquivalente Abgangsgruppen.

Der Ausdruck »pharmazeutisch annehmbares Säureadditionssalz« umfaßt pharmazeutisch annehmbare Salze der Verbindung der Formel I sowohl mit anorganischen Säuren, wie Salzsäure, Bromwasserstoff, Phosphorsäure und Schwefelsäure als auch mit organischen Säuren, wie Maleinsäure, Citronensäure, Essigsäure, Bernsteinsäure, Apfelsäure, Weinsäure, Camphersulfonsäure, Mandelsäure, Fumarsäure, Methansulfonsäure und p-Toluolsulfonsäure. Die Herstellung der pharmazeutisch annehmbaren Säureadditionssalze erfolgt nach an sich bekannten und jedem Fachmann geläufigen Methoden.

Die Verbindung der Formel I besitzt ein asymmetrisches Kohlenstoffatom; die Erfindung umfaßt sowohl die optisch einheitlichen Formen als auch Gemische davon (insbesondere das Racemat). Die Spaltung des Racemates kann nach an sich bekannten Methoden durchgeführt werden, beispielsweise durch fraktionierte Kristallisation eines Säureadditionssalzes der Verbindung der Formel I mit einer optisch aktiven Säure (z. B. mit Weinsäure, Camphersulfonsäure oder Mandelsäure). Die optisch einheitlichen Formen können aber auch erhalten werden, indem man in der nachfolgend beschriebenen Verfahrensvariante c) einen optisch einheitlichen Ausgangsstoff verwendet.

Ein ganz besonders bevorzugtes, pharmazeutisch annehmbares Säureadditionssalz der Verbindung der Formel I ist das 1-[2-(4,5,10,11-Tetrahydro-1H-dibenzo[a,d]cyclohepten-5-yl)äthyl]pyrrolidin-maleat.

Die Verbindung der Formel I, d. h. 1-[2-(4,5,10,11-Tetrahydro-1H-dibenzo[a,d]cyclohepten-5-yl)äthyl]pyrrolidin, und ihre pharmazeutisch annehmbaren Säureadditionssalze können dadurch hergestellt werden, daß man

a) 1-[2-(10,11-Dihydro-5H-dibenzo[a,d]cyclohepten-5-yl)äthyl]pyrrolidin 1,4-reduziert oder

b) 1,4,10,11-Tetrahydro-5H-dibenzo[a,d]cyclohepten in Gegenwart einer starken Base mit einer Verbindung der allgemeinen Formel II

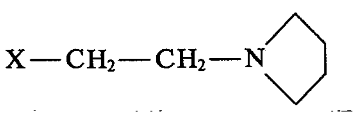

(II)

worin X eine Abgangsgruppe bedeutet,

umsetzt, oder

c) eine Verbindung der allgemeinen Formel III

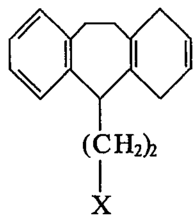

(III)

worin X obige Bedeutung besitzt,

mit Pyrrolidin umsetzt, und

d) das so erhaltene 1-[2-(4,5,10,11-Tetrahydro-1H-dibenzo[a,d]cyclohepten-5-yl)äthyl]pyrrolidin als freie Base oder als pharmazeutisch annehmbares Säureadditionssalz isoliert.

Nach Variante a) des erfindungsgemäßen Verfahrens kann die Verbindung der Formel I hergestellt werden, indem man in 1-[2-(10,11-Dihydro-5H-dibenzo[a,d]cyclohepten-5-yl)äthyl]pyrrolidin einen der beiden aromatischen Ringe 1,4-reduziert. Als Reduktionsmittel kann man beispielsweise ein Alkalimetall, wie Lithium, Natrium oder Kalium, verwenden, wobei man als Lösungsmittel flüssiges Ammoniak oder ein für solche Reduktionen geeignetes Amin, wie Methylamin, Äthylamin oder Dimethylamin, einsetzt. Zweckmäßigerweise arbeitet man in Gegenwart eines Protonendonators und eines Lösungsvermittlers. Als Protonendonatoren kommen in erster Linie Alkohole, wie Methanol, Äthanol, Propanol, Isopropanol, Butanol, t-Butanol, 1,1-Dimethylpropanol, Äthylenglykolmonomethyläther oder Propylenglykolmonomethyläther, in Frage. Geeignete Lösungsvermittler sind beispielsweise Äther, wie Diäthyläther, t-Butylmethyläther, Tetrahydrofuran, Dioxan, Äthylenglykoldimethyläther oder Diglyme. Die Reaktionstemperatur liegt je nach verwendetem Lösungsmittel in einem Bereich von etwa —50° C bis Siedetemperatur des Reaktionsgemisches.

Die 1,4-Reduktion kann beispielsweise so durchgeführt werden, daß man eine Lösung von 1-[2-(10,11-Dihydro-5H-dibenzo[a,d]cyclohepten-5-yl)äthyl]pyrrolidin in einer Mischung aus dem Lösungsmittel, vorzugsweise siedendem Ammoniak, dem Lösungsvermittler, vorzugsweise trockenem Tetrahydrofuran, und dem Protonendonator, vorzugsweise trockenem t-Butanol oder Äthanol, vorbereitet, und diese Lösung mit dem Alkalimetall, vorzugsweise Lithium oder Natrium, versetzt.

Man kann aber ohne weiteres auch umgekehrt verfahren, d. h. man kann eine Lösung des Alkalimetalls im Lösungsmittel vorbereiten, und diese mit einer Lösung von 1-[2-(10,11-Dihydro-5H-dibenzo[a,d]cyclohepten-5-yl)äthyl]pyrrolidin in einer Mischung aus Lösungsvermittler und Protonendonator versetzen.

Eine weitere Ausführungsform besteht darin, daß man den Protonendonator erst nach beendeter 1,4-Reduktion dem Reaktionsgemisch zugibt. In diesem Fall eignen sich als Protonendonatoren auch saure Ammoniumsalze, wie Ammoniumchlorid.

Schließlich kann die gewünschte 1,4-Reduktion auch elektrochemisch durchgeführt werden. Die elektrochemische 1,4-Reduktion kann in einer ungeteilten oder in einer geteilten Zelle durchgeführt werden, wobei die ungeteilte Zelle bevorzugt wird. Das Kathodenmaterial ist nicht kritisch, und man kann demnach Platin, Graphit, Quecksilber, Blei, Nickel oder Aluminium verwenden. Als Kathodenmaterial verwendet man vorzugsweise Platin. Das bevorzugte Anodenmaterial ist Platin, wobei auch Blei oder Graphit oder andere nicht korrodierende Werkstoffe verwendet werden können. Als Lösungsmittel kommen Amine, wie Methylamin, Propylamin oder Äthylendiamin in Frage. Gegebenenfalls können auch Lösungsvermittler, wie Tetrahydrofuran und Diäthylenglykoldimethyläther, und/oder Protonendonatoren, wie Äthanol und t-Butanol, eingesetzt werden. Für den vorliegenden Verfahrensaspekt geeignete Leitsalze sind beispielsweise Lithiumchlorid, Natriumchlorid und Tetrabutylammoniumchlorid. Die Reaktionstemperatur ist nicht kritisch, man kann demnach in Abhängigkeit vom eingesetzten Lösungsmittel in einem Temperaturbereich von etwa —20 bis etwa 100° C arbeiten.

In einer besonders bevorzugten Ausführungsform verwendet man Methylamin als Lösungsmittel, Lithiumchlorid als Leitsalz und Platin als Anoden- und Kathodenmaterial und arbeitet bei einer Temperatur von etwa —10° C.

Nach Variante b) des erfindungsgemäßen Verfahrens kann die Verbindung der Formel I hergestellt werden, indem man 1,4,10,11-Tetrahydro-5H-dibenzo[a,d]cyclohepten in Gegenwart einer starken Base mit einer Verbindung der allgemeinen Formel II umsetzt. Zweckmäßigerweise wird das 1,4,10,11-Tetrahydro-5H-dibenzo[a,d]cyclohepten in einem inerten organischen Lösungsmittel, beispielsweise in einem Äther, wie Tetrahydrofuran, Dioxan, Diäthyläther, Dimethoxyäthan, Diglyme oder t-Butylmethyläther, oder in einer Mischung davon mit Alkanen, wie z. B. Pentan, Hexan und Heptan, mit einer starken Base, beispielsweise mit einer Alkyl- oder Aryllithiumverbindung, wie n-Butyllithium, Methyllithium und Phenyllithium, oder mit einem Alkalimetallamid, wie Lithiumdiisopropylamid und Natriumamid, oder mit Natriumhydrid, in das entsprechende Anion übergeführt, und dieses mit einer

Verbindung der allgemeinen Formel II umgesetzt. Je nach eingesetzter Base kann man bei Temperaturen von etwa −70° C bis etwa Raumtemperatur arbeiten.

Nach Variante c) des erfindungsgemäßen Verfahrens kann die Verbindung der Formel I hergestellt werden, indem man eine Verbindung der allgemeinen Formel III mit Pyrrolidin umsetzt. Diese Reaktion erfolgt zweckmäßigerweise in einem inerten organischen Lösungsmittel in Gegenwart eines säurebindenden Mittels. Für den vorliegenden Verfahrensaspekt geeignete Lösungsmittel sind beispielsweise Äther, wie Diäthyläther, t-Butylmethyläther, Tetrahydrofuran oder Äthylenglycoldimethyläther, Alkohole, wie Äthanol oder Äthylenglykol, und überschüssiges Pyrrolidin. Als säurebindende Mittel kommen anorganische Basen, wie Kalium- und Natriumcarbonat, oder organische Basen, wie Triäthylamin und Chinuclidin, oder überschüssiges Pyrrolidin in Frage. In einer bevorzugten Ausführungsform verwendet man überschüssiges Pyrrolidin als Lösungsmittel und gleichzeitig als säurebindendes Mittel. Die Reaktionstemperatur kann in einem Bereich von etwa 0° C bis Siedetemperatur des Reaktionsgemisches variieren und ist natürlich abhängig von der Reaktivität der mit X bezeichneten Abgangsgruppe.

Erfindungsgemäß wird die Verbindung der Formel I als freie Base oder als pharmazeutisch annehmbares Säureadditionssalz isoliert. Die Isolierung der freien Base oder ihrer pharmazeutisch annehmbaren Säureadditionssalze erfolgt nach an sich bekannten und jedem Fachmann geläufigen Methoden, beispielsweise durch Extraktions- oder Filtrationstechniken, durch gegebenenfalls fraktionierte Kristallisation, durch chromatographische Methoden, wie Gaschromatographie und Hochdruck-Flüssigkeits-Chromatographie, oder durch Destillation, oder durch geeignete Kombination mehrerer dieser an sich bekannten Methoden.

Das als Ausgangsmaterial verwendete 1-[2-(10,11-Dihydro-5H-dibenzo[a,d]cyclohepten-5-yl)äthyl]pyrrolidin ist ein bekannter Stoff. Gleichwohl enthalten verschiedene der weiter unten folgenden Beispiele detaillierte Angaben betreffend die Herstellung dieses Stoffes.

Das als Ausgangsstoff verwendete 1,4,10,11-Tetrahydro-5H-dibenzo[a,d]cyclohepten wird zweckmäßigerweise aus dem bekannten 10,11-Dihydro-5H-dibenzo[a,d]cyclohepten hergestellt, und zwar durch entsprechende Reduktion in Analogie zur weiter oben beschriebenen Verfahrensvariante a).

Die als Ausgangsstoffe verwendeten Verbindungen der allgemeinen Formel III können hergestellt werden, indem man 10,11-Dihydro-5H-dibenzo[a,d]cyclohepten-5-äthanol in Analogie zur weiter oben beschriebenen Verfahrensvariante a) entsprechend reduziert und die Hydroxygruppe im erhaltenen 1,4,10,11-Tetrahydro-5H-dibenzo[a,d]cyclohepten-5-äthanol in eine Abgangsgruppe umwandelt. Diese Umwandlung erfolgt nach an sich bekannten und jedem Fachmann geläufigen Methoden; beispielsweise durch Behandeln von 1,4,10,11-Tetrahydro-5H-dibenzo[a,d]cyclohepten-5-äthanol mit einem Halogenierungsmittel, wie Thionylchlorid, Phosphoroxychlorid, Phosphorpentachlorid, Tetrabromkohlenstoff/Triphenylphosphin, Jod/roter Phosphor oder dergleichen, oder mit einem reaktiven Sulfonsäurederivat, wie Mesylchlorid, Tosylchlorid, Brosylchlorid oder Benzolsulfonsäurechlorid, und erwünschtenfalls Austauschen des Sulfonsäurerestes gegen ein Halogenatom nach an sich bekannten Methoden. 10,11-Dihydro-5H-dibenzo[a,d]cyclohepten-5-äthanol ist ein bekannter Stoff; gleichwohl enthalten einige der weiter unten folgenden Beispiele detaillierte Angaben betreffend die Herstellung dieses Stoffes.

Die Verbindungen der allgemeinen Formel III und das 1,4,10,11-Tetrahydro-5H-dibenzo[a,d]cyclohepten sind ebenfalls Gegenstand der Erfindung.

Überraschenderweise hat es sich gezeigt, daß die erfindungsgemäßen Produkte die $H_1$-Wirkung des Histamins zu hemmen vermögen. Die Verbindung der Formel I und ihre pharmazeutisch annehmbaren Säureadditionssalze sind somit wertvolle Histamin-$H_1$ antagonistische Wirkstoffe und eignen sich insbesondere zur Bekämpfung bzw. Verhütung von allergischen Reaktionen, wie beispielsweise Urtikaria, Heuschnupfen, Anaphylaxie und Arzneimittelüberempfindlichkeit. Diese Histamin-$H_1$ antagonistischen Eigenschaften können wie nachfolgend beschrieben an männlichen und weiblichen, 240 bis 300 g schweren Meerschweinchen (SPF, Füllinsdorf) ermittelt werden:

Den Versuchstieren (10 pro Dosis) wird während 24 Stunden vor Versuchsbeginn das Futter entzogen, wobei Wasser ad libitum erhältlich ist. Eine Stunde nach oraler Verabreichung einer Lösung der Testsubstanz (10 ml/kg) erhalten die Versuchstiere eine letale Dosis von Histamin-dihydrochlorid (10 mg/kg s.c.). Ungeschützte Tiere, d. h. nur mit Histamin-dihydrochlorid behandelte Tiere, sterben innerhalb einer Stunde. Nach Auszählen der überlebenden, geschützten Tiere wird die $ED_{50}$ nach der Probitmethode ermittelt. Die $ED_{50}$ ist diejenige Dosis, die notwendig ist, um 50% der mit der Testsubstanz behandelten Tiere vor dem Tod zu schützen.

Für 1-[2-(4,5,10,11-Tetrahydro-1H-dibenzo[a,d]cyclohepten-5-yl)äthyl]pyrrolidin-maleat wurde eine $ED_{50}$ von 0,18 mg/kg p.o. und eine $DL_{50}$ von 200 mg/kg (nach oraler Verabreichung an 5 aufeinanderfolgenden Tagen an Mäusen) ermittelt.

Die Verbindung der Formel I und ihre pharmazeutisch annehmbaren Säureadditionssalze können als Heilmittel, z. B. in Form pharmazeutischer Präparate, Verwendung finden. Die pharmazeutischen Präparate können oral, z. B. in Form von Tabletten, Lacktabletten, Dragées, Hart- und Weichgelatinekapseln, Lösungen, Emulsionen oder Suspensionen, verabreicht werden. Die Verabreichung kann aber auch rektal, z. B. in Form von Suppositorien, oder parenteral, z. B. in Form von Injektionslösungen, erfolgen.

Zur Herstellung von pharmazeutischen Präparaten können die erfindungsgemäßen Produkte mit pharmazeutisch inerten, anorganischen oder organischen Trägern verarbeitet werden. Als solche Träger kann man für Tabletten, Lacktabletten, Dragées und Hartgelatinekapaseln beispielsweise Lactose, Maisstärke oder Derivate davon, Talk, Stearinsäure oder deren Salze verwenden. Für Weichgelatinekapseln eignen sich als Träger beispielsweise pflanzliche Öle, Wachse, Fette, halbfeste und flüssige Polyole; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker oder Glukose. Für Injektionslösungen eignen sich als Träger beispielsweise Wasser, Alkohole, Polyole, Glyzerin oder pflanzliche Öle. Für Suppositorien eignen sich als Träger beispielsweise natürliche oder gehärtete Öle, Wachse, Fette, halbflüssige oder flüssige Polyole.

Die pharmazeutischen Präparate können daneben noch Konservierungsmittel, Lösungsvermittler, Stabilisierungsmittel, Netzmittel, Emulgiermittel, Süßmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt sind Arzneimittel, enthaltend die Verbindung der Formel I oder ein pharmazeutisch annehmbares Säureadditionssalz davon, ebenfalls Gegenstand der Erfindung, weiterhin auch ein Verfahren zur Herstellung solcher Arzneimittel, welches dadurch gekennzeichnet ist, daß man die Verbindung der Formel I oder ein pharmazeutisch annehmbares Säureadditionssalz davon und gegebenenfalls einen oder mehrere andere therapeutisch wertvolle Stoffe in eine galenische Darreichungsform bringt. Wie eingangs erwähnt, können die Verbindungen der Formel I und ihre pharmazeutisch annehmbaren Säureadditionssalze bei der Bekämpfung bzw. Verhütung von Krankheiten verwendet werden; insbesondere können sie bei der Bekämpfung bzw. Verhütung von allergischen Reaktionen, wie Urtikaria, Heuschnupfen, Anaphylaxie und Arzneimittelüberempfindlichkeit verwendet werden. Die Dosierung kann innerhalb weiter Grenzen variieren und ist natürlich in jedem einzelnen Fall den individuellen Gegebenheiten anzupassen. Im allgemeinen dürfte bei oraler Verabreichung eine Tagesdosis von etwa 10 mg bis 150 mg angemessen sein.

In den nachfolgenden Beispielen, welche die Erfindung näher erläutern, ihren Umfang jedoch in keiner Weise beschränken sollen, sind sämtliche Temperaturen in Celsiusgraden angegeben.

## Beispiel 1

a) Eine Mischung aus 208 g 10,11-Dihydro-5H-dibenzo[a,d]cyclohepten-5-on, 2 l Äthanol, 200 g Natriumhydroxid und 300 g Zinkpulver wird unter Rühren während 2 Stunden unter Rückfluß zum Sieden erhitzt. Das noch warme Reaktionsgemisch wird unter Nachwaschen mit etwa 1 l Methanol über Kieselgur filtriert. Nach Einengen der gelben Lösung auf ca. 1 l wird der erhaltene dicke Brei zwischen 2 l Chloroform und 1 l Wasser verteilt. Die alkalische wäßrige Phase wird unter Eiskühlung mit ca. 400 ml Salzsäure konz. auf pH 3 bis 4 gestellt und mit 1 l Chloroform ausgeschüttelt. Die vereinigten Chloroformauszüge werden bis zur neutralen Reaktion mit Wasser gewaschen und anschließend über Magnesiumsulfat in Gegenwart von wenig Aktivkohle getrocknet. Nach dem Filtrieren und Einengen der klaren, leicht gelblichen Lösung erhält man 10,11-Dihydro-5H-dibenzo[a,d]cyclohepten-5-ol, als fast weiße, kristalline Masse mit einem Schmelzpunkt von 89—91°.

Durch Umkristallisieren einer Probe dieses Materials aus Äther/Petroläther erhält man reines Produkt vom Schmelzpunkt 92—93°.

b) Zu einer Lösung von 201,5 g rohem 10,11-Dihydro-5H-dibenzo[a,d]cyclohepten-5-ol in 700 ml trockenem Benzol tropft man über einen Zeitraum von 40 Minuten 350 ml Thionylchlorid bei einer Temperatur von 30 bis 40° zu. Anschließend wird noch während 1,5 Stunden unter Rückfluß zum Sieden erhitzt und im Vakuum eingeengt. Der Rückstand wird 2 bis 3mal mit je 300 ml Benzol verdünnt und jeweils wieder eingedampft. Man erhält 5-Chlor-10,11-dihydro-5H-dibenzo[a,d]cyclohepten als beige kristalline Masse vom Schmelzpunkt 99—101°, welche ohne weitere Reinigung in die nächste Stufe eingesetzt wird. Durch Umkristallisieren aus Tetrachlorkohlenstoff erhält man Material vom Schmelzpunkt 104—105°.

c) Ein Gemisch aus 11,4 g Magnesiumspänen, 151,5 g Malonsäure-diäthylester und 300 ml trockenem Äthanol wird unter Argon über einen Zeitraum von 30 Minuten auf 60° erwärmt. Nach Abklingen der heftigen Reaktion wird noch 1 Stunde unter Rückfluß zum Sieden erhitzt und dann im Vakuum eingeengt. Man versetzt den Rückstand zweimal mit je 300 ml trockenem Benzol und dampft jeweils gut ein. Der erhaltene Rückstand wird unter Argon in 350 ml trockenem Tetrahydrofuran gelöst. Dazu tropft man bei Raumtemperatur über einen Zeitraum von 20 Minuten eine Lösung von 195,5 g 5-Chlor-10,11-dihydro-5H-dibenzo[a,d]cyclohepten in 500 ml trockenem Tetrahydrofuran zu. Das Reaktionsgemisch wird unter Rühren über Nacht unter Rückfluß zum Sieden erhitzt und dann im Vakuum eingedampft. Der erhaltene Rückstand wird zwischen 2,0 l Toluol und 500 ml Eiswasser verteilt. Die organische Phase wird nacheinander zweimal mit je 500 ml 1N Salzsäure, zweimal mit je 500 ml Wasser, zweimal mit je 250 ml gesättigter Natriumbicarbonatlösung und mehrmals mit Wasser gewaschen,

über Magnesiumsulfat getrocknet und im Vakuum eingedampft. Man erhält 10,11-Dihydro-5H-dibenzo[a,d]cyclohepten-5-malonsäure-diäthylester als oranges Öl.

d) Eine Lösung von 305 g rohem 10,11-Dihydro-5H-dibenzo[a,d]cyclohepten-5-malonsäure-diäthylester und 138 g Kaliumhydroxid in 1,5 l Äthanol und 400 ml Wasser wird unter Rühren während 1,5 Stunden unter Rückfluß zum Sieden erhitzt und anschließend im Vakuum eingedampft. Der erhaltene Rückstand wird zwischen 1 l Äther und 1 l Eiswasser verteilt. Die wäßrige, alkalische Phase wird noch mit 500 ml Äther ausgeschüttelt, unter Eiskühlung mit etwa 220 ml Salzsäure konz. sauer gestellt und mit 2 l Äther ausgeschüttelt. Die ätherische Phase wird dreimal mit je 500 ml Wasser gewaschen; die Waschwasser werden mit 1 l Äther zurückextrahiert. Die vereinigten Ätherauszüge werden noch drei- bis viermal mit gesättigter Kochsalzlösung gewaschen, über Magnesiumsulfat getrocknet und auf ein Volumen von ca. 400 ml eingeengt. Man verdünnt mit 300 ml Petroläther,kühlt die erhaltene klare Lösung in einem Eisbad und läßt über Nacht bei 4° stehen. Das auskristallisierte Material wird unter Nachwaschen mit Petroläther abgenutscht und im Vakuumtrockenschrank bei 50° getrocknet. Man erhält 10,11-Dihydro-5H-dibenzo[a,d]cyclohepten-5-malonsäure vom Schmelzpunkt 183° (unter Decarboxylierung).

e) 200 g 10,11-Dihydro-5H-dibenzo[a,d]cyclohepten-5-malonsäure werden während 30 Minuten unter Rühren auf 180° erhitzt. Nach dem Abkühlen auf 120° versetzt man mit etwa 1 l Benzol und erhitzt unter Rühren und Rückfluß zum Sieden, bis alles auskristallisierte Material wieder gelöst ist. Anschließend versetzt man mit 500 ml Hexan, kühlt unter Rühren und Eiskühlung auf ca. 10° ab, läßt über Nacht in der Kälte stehen und nutscht anschließend den erhaltenen Kristallbrei ab. Nach Waschen mit Petroläther und Trocknen im Vakuumtrockenschrank bei 50° erhält man 10,11-Dihydro-5H-dibenzo[a,d]cyclohepten-5-essigsäure als beige Kristalle vom Schmelzpunkt 163—165°.

f) Eine Mischung aus 160 g 10,11-Dihydro-5H-dibenzo[a,d]cyclohepten-5-essigsäure und 230 ml Thionylchlorid wird während 2,5 Stunden unter Rückfluß zum Sieden erhitzt. Nach Einengen des Reaktionsgemisches im Vakuum versetzt man den Rückstand zweimal mit je 300 ml trockenem Benzol und dampft jeweils zur Trockne ein. Man erhält 10,11-Dihydro-5H-dibenzo[a,d]cyclohepten-5-essigsäurechlorid als rotbraunes Öl.

g) Eine Mischung aus 270 ml Pyrrolidin und 400 ml trockenem Benzol wird unter einer Argonatmosphäre bei 0 bis 20° über einen Zeitraum von 1 Stunde tropfenweise mit einer Lösung von 174,4 g 10,11-Dihydro-5H-dibenzo[a,d]cyclohepten-5-essigsäurechlorid in 400 ml trockenem Benzol versetzt. Man erhitzt das Reaktionsgemisch noch 1 Stunde unter Rückfluß zum Sieden, läßt auf etwa 30° abkühlen und gießt auf ca. 2 l Eiswasser. Die wäßrige Phase wird abgetrennt und mit 1 l Benzol ausgeschüttelt. Die vereinigten organischen Auszüge werden nacheinander zweimal mit je 500 ml 2N Natronlauge, 500 ml Wasser, 500 ml 2N Salzsäure und zwei- bis dreimal mit je 500 ml gesättigter Kochsalzlösung gewaschen, über Magnesiumsulfat getrocknet und eingedampft. Nach Aufnehmen des Rückstandes in 500 ml heißem Aceton läßt man auf ca. 50° abkühlen und versetzt mit 1,2 l Petroläther. Man läßt über Nacht bei 4° stehen und erhält nach Abfiltrieren des auskristallisierten Materials 1-[(10,11-Dihydro-5H-dibenzo[a,d]cyclohepten-5-yl)acetyl]-pyrrolidin vom Schmelzpunkt 130—131°. Aus der Mutterlauge erhält man noch eine zweite Portion des gewünschten Amids mit einem Schmelzpunkt von 128—130°.

h) Eine unter Argon gerührte Mischung von 28,5 g Lithiumaluminiumhydrid und 300 ml trockenem Dioxan versetzt man über einen Zeitraum von 1,25 Stunden tropfenweise mit einer Lösung von 230 g 1-[(10,11-Dihydro-5H-dibenzo[a,d]cyclohepten-5-yl)acetyl]pyrrolidin in 700 ml trockenem Dioxan, wobei man unter Rückfluß zum Sieden erhitzt. 10 Minuten nach Beendigung der Zugabe kühlt man auf ca. 10—15° ab und zerstört überschüssiges Lithiumaluminiumhydrid durch vorsichtige Zugabe von 200 ml Essigester, wobei die Temperatur auf ca. 40° ansteigt. Das Reaktionsgemisch wird anschließend auf ca. 10° abgekühlt und unter guter Kühlung durch langsames Zutropfen von ca. 250 ml Wasser hydrolysiert. Man nutscht unter Nachwaschen mit ca. 200 ml Chloroform ab und dampft im Vakuum ein. Das zurückbleibende braune Öl wird in 1 l Äther aufgenommen und nacheinander mit 300 ml und 150 ml 2N Salzsäure und zweimal mit je 100 ml Wasser ausgeschüttelt. Die saure wäßrige Phase wird unter Eiskühlung mit ca. 100 ml 28proz. Natronlauge basisch gestellt und nacheinander mit 500 ml und zweimal mit je 300 ml Hexan ausgeschüttelt. Die organischen Auszüge werden zweimal mit je 250 ml gesättigter Kochsalzlösung und zweimal mit je 250 ml destilliertem Wasser gewaschen, über Magnesiumsulfat in Gegenwart einer Spatelspitze Aktivkohle getrocknet und im Vakuum eingedampft. Nach Trocknen des Rückstandes im Hochvakuum bei 40° erhält man 1-[2-(10,11-Dihydro-5H-dibenzo[a,d]cyclohepten-5-yl)-äthyl]pyrrolidin als hellgelbes Öl, das langsam kristallisiert; Smp. 51—53°. Durch Destillation der rohen Base unter Argon über einen Hickmann-Aufsatz bei 163—165°/0,09 Torr erhält man reines Material vom Schmelzpunkt 53,5—54°.

i) Zu einer Mischung aus 2,6 l trockenem Ammoniak und 1,6 l trockenem Tetrahydrofuran gibt man bei —50° unter einer Argonatmosphäre 10,4 g Lithium. Nach 2,5 Minuten versetzt man die tiefblaue Reaktionslösung unter gutem Rühren bei —50 bis —43° über einen Zeitraum von 6 Minuten mit einer auf —50° vorgekühlten Lösung von 150 g 1-[2-(10,11-Dihydro-5H-dibenzo[a,d]cyclohepten-5-yl)-äthyl]pyrrolidin und 351 g t-Butanol in 1,8 l trockenem Tetrahydrofuran. Nach beendeter Zugabe wird das dunkelblaue Reaktionsgemisch bei —43° bis —39° bis zur Entfärbung gerührt (ca. 10 Minuten). Nach weiteren 2 Minuten versetzt man mit 12,0 g Natriumbenzoat und entfernt anschließend das

Ammoniak. Der Rückstand wird anschließend sechsmal mit je 500 ml gesättigter Kochsalzlösung gewaschen, über Magnesiumsulfat getrocknet und im Vakuum eingedampft. Das erhaltene hellbraune Öl wird durch Säulenchromatographie an 500 g Aluminiumoxid (Aktivitätsstufe II, neutral) unter Eluieren mit Benzol gereinigt, wobei man rohes 1-[2-(4,5,10,11-Tetrahydro-1H-dibenzo[a,d]cyclohepten-5-yl)äthyl]pyrrolidin als hellgelbes Öl erhält, das beim Stehen langsam kristallisiert.

j) 660 g rohes 1-[2-(4,5,10,11-Tetrahydro-1H-dibenzo[a,d]cyclohepten-5-yl)äthyl]pyrrolidin werden unter einer Argonatmosphäre unter leichtem Erwärmen (auf ca. 40°) in 3,0 l Aceton gelöst. Die erhaltene Lösung wird auf ca. 10° abgekühlt und unter Rühren mit einer auf ca. 10° vorgekühlten Lösung von 264,0 g Maleinsäure in 1,6 l Aceton versetzt und unter Durchleiten von Argon mit 4,0 l n-Hexan verdünnt. Nach 15 Stunden bei Raumtemperatur und unter Lichtausschluß wird der entstandene Kristallbrei abgenutscht, zweimal mit je 1,0 l Petroläther gewaschen und im Vakuumtrockenschrank während 20 Stunden bei Raumtemperatur getrocknet. Man erhält 1-[2-(4,5,10,11-Tetrahydro-1H-dibenzo[a,d]cyclohepten-5-yl)äthyl]pyrrolidin-maleat vom Schmelzpunkt 127—129°. Aus der Mutterlauge erhält man durch Einengen auf ca. 1 l noch eine zweite Portion vom Schmelzpunkt 123—125°.

k) Eine Mischung aus 8 g 1-[2-(4,5,10,11-Tetrahydro-1H-dibenzo[a,d]cyclohepten-5-yl)äthyl]pyrrolidin-maleat, 20 ml 3N Natronlauge und 50 ml Äther wird so lange geschüttelt, bis zwei klare Phasen entstehen. Nach Abtrennen der wäßrigen Phase wird die ätherische Lösung über Magnesiumsulfat getrocknet und eingedampft. Man erhält kristallines 1-[2-(4,5,10,11-Tetrahydro-1H-dibenzo[a,d]cyclohepten-5-yl)äthyl]pyrrolidin vom Schmelzpunkt 57—58,5°, das nach Umkristallisieren aus tiefsiedendem Petroläther bei —25° bei 61—63° schmilzt.

l) Durch Hochdruck-Flüssigkeits-Chromatographie von 10 g des obigen Materials an zwei in Serie geschalteten, im Handel erhältlichen, vorbepackten Kieselgelsäulen (5,7 × 30 cm) erhält man nach neunmaligem Recycling unter Eluieren mit 10% Tetrahydrofuran und 0,5% Isopropylamin in n-Hexan und Umkristallisieren aus Petroläther (tiefsiedend) bei —25° reines 1-[2-(4,5,10,11-Tetrahydro-1H-dibenzo[a,d]cyclohepten-5-yl)äthyl]pyrrolidin vom Schmelzpunkt 63—65°.

Nach Umkristallisieren aus Aceton/n-Hexan hat das aus obigem Material hergestellte Maleinsäuresalz einen Schmelzpunkt von 129—131°.

## Beispiel 2

a) Eine bei —40° gerührte Mischung aus 1,28 l trockenem Tetrahydrofuran und 2,1 l flüssigem, trockenem Ammoniak wird mit 8,6 g Lithiumdraht versetzt. Man rührt während 2 Minuten und gibt anschließend eine auf —40° vorgekühlte Lösung von 80,0 g 10,11-Dihydro-5H-dibenzo[a,d]cyclohepten in 1,4 l trockenem Tetrahydrofuran und 280 g t-Butanol über einen Zeitraum von 6 Minuten hinzu. Nach Farbumschlag von blau zu farblos, versetzt man mit 6,0 g Natriumbenzoat und dampft das Ammoniak ab. Das Reaktionsgemisch wird dreimal mit je 500 ml Wasser gewaschen. Die vereinigten Waschwasser werden zweimal mit je 1,0 l Äther extrahiert. Die organischen Phasen werden vereinigt, mit gesättigter Kochsalzlösung gewaschen, über Magnesiumsulfat getrocknet und eingeengt. Der ölige Rückstand wird durch fraktionierte Kristallisation aus Petroläther (tiefsiedend) bei —25° gereinigt. Man erhält 1,4,10,11-Tetrahydro-5H-dibenzo[a,d]cyclohepten als weiße Kristalle vom Schmelzpunkt 42,5—43°.

b) 1,0 g 1,4,10,11-Tetrahydro-5H-dibenzo[a,d]cyclohepten in 20 ml trockenem Tetrahydrofuran werden zwischen —70 und —60° tropfenweise mit 9,0 ml einer etwa 0,85 M Lösung von n-Butyllithium in Hexan versetzt. Man rührt während 60 Minuten bei —70° und während 30 Minuten bei —30°, tropft eine Lösung von 2,1 g 2-Chloräthylpyrrolidin in 5 ml trockenem Tetrahydrofuran bei —30° hinzu und rührt anschließend noch während 30 Minuten bei —30° 3 Stunden bei 0 bis 5° und 30 Minuten bei Raumtemperatur. Nach Eindampfen der Reaktionsmischung wird der verbleibende Rückstand in Toluol gelöst und mit Wasser ionenfrei gewaschen. Die organische Phase wird unter Eluieren mit Toluol über Aluminiumoxid (Aktivitätsstufe II, neutral) filtriert. Man erhält rohes 1-[2-(4,5,10,11-Tetrahydro-1H-dibenzo[a,d]cyclohepten-5-yl)äthyl]pyrrolidin.

Durch präparative Gaschromatographie an einer 2 m langen (Durchmesser: 40 mm) mit 4% Carbowax 20 M (Polyäthylenglykol) beladenen Kolonne [Trägermaterial: Chromosorb G NAW (Diatomeenerde)] erhält man bei einer Ofentemperatur von 220° unter Verwendung von Helium als Trägergas reines 1-[2-(4,5,10,11-Tetrahydro-1H-dibenzo[a,d]cyclohepten-5-yl)äthyl]pyrrolidin vom Schmelzpunkt 58—60°.

## Beispiel 3

a) 1,0 g 10,11-Dihydro-5H-dibenzo[a,d]cyclohepten in 25 ml trockenem Tetrahydrofuran werden bei —70° tropfenweise mit 6,8 ml einer etwa 1,14 M Lösung von n-Butyllithium in Hexan versetzt. Man rührt während 60 Minuten bei —70°, tropft anschließend bei —30° 1,1 g 2Chloräthylpyrrolidin in 2,5 ml trockenem Tetrahydrofuran hinzu und rührt während 1 Stunde bei —30° und während 3 Stunden bei 0 bis 5°. Nach Eindampfen der Reaktionsmischung wird der verbleibende Rückstand in Toluol gelöst und mit Wasser gewaschen. Die organische Phase wird unter Eluieren mit Toluol über 10 g Aluminiumoxid

(Aktivitätsstufe II, neutral) filtriert. Man erhält rohes 1-[2-(10,11-Dihydro-5H-dibenzo[a,d]cyclohepten-5-yl)äthyl]pyrrolidin als gelbes Öl.

Nach Reinigung dieses Produkts gemäß Angaben in Beispiel 1h) erhält man daraus nach den Angaben in den Beispielen 1i) bis 1l) reines 1-[2-(4,5,10,11-Tetrahydro-1H-dibenzo[a,d]cyclohepten-5-yl)äthyl]pyrrolidin.

Beispiel 4

a) Eine unter Argon gerührte Lösung von 58,3 g Dibenzosuberan in 750 ml Tetrahydrofuran wird im Eisbad auf etwa 10° abgekühlt und tropfenweise mit 190 ml einer etwa 2 M Lösung von Butyllithium in Hexan versetzt. Die dunkelrote Reaktionslösung wird während 2 Stunden unter Rückfluß zum Sieden erhitzt. In die auf etwa 10° abgekühlte Reaktionslösung leitet man während 15 Minuten Äthylenoxid ein, rührt anschließend während 1 Stunde bei Raumtemperatur und erhitzt schließlich noch während 30 Minuten unter Rückfluß zum Sieden. Das erkaltete Reaktionsgemisch wird auf 300 ml eiskalte 3 N Salzsäure gegossen und zweimal mit je 2 l Äther ausgeschüttelt. Die organischen Auszüge werden mit 500 ml 2N Kaliumbicarbonatlösung gewaschen, vereinigt, über Magnesiumsulfat getrocknet und eingedampft. Der Rückstand wird dreimal mit je 50 ml Pentan ausgekocht. Man erhält 10,11-Dihydro-5H-dibenzo[a,d]cyclohepten-5-äthanol in Form eines sehr viskosen, gelblichen Öls, Rf: 0,25 (Toluol/Essigester, 9 : 1).

b) Eine unter Argon gerührte Mischung aus 80,0 g 10,11-Dihydro-5H-dibenzo[a,d]cyclohepten-5-äthanol, 195,7 ml trockenem Äthanol, 2,0 l trockenem Tetrahydrofuran und 3,2 l trockenem, destilliertem Ammoniak wird bei —30 bis —31° mit 25,09 g Natrium (3 bis 4 Stücke) versetzt. Nach Abklingen der exothermen Reaktion wird das Reaktionsgemisch noch etwa 10 Minuten gerührt und anschließend portionenweise mit insgesamt 60 g Ammoniumchlorid versetzt. Nach Entfernen des Ammoniaks wird die verbleibende Suspension dreimal mit je 800 ml gesättigter Ammoniumchloridlösung gewaschen. Die wäßrigen Auszüge werden zweimal mit je 1,5 l Äther ausgeschüttelt. Die organischen Phasen werden mit 800 ml Wasser gewaschen, vereinigt, getrocknet und im Vakuum eingedampft. Man erhält rohes 4,5,10,11-Tetrahydro-1H-dibenzo[a,d]cyclohepten-5-äthanol in Form eines Öles, das ohne weitere Reinigung in die nächste Stufe eingesetzt wird.

c) Zu einer bei Raumtemperatur gerührten Lösung von 87,5 g rohem 4,5,10,11-Tetrahydro-1H-dibenzo[a,d]cyclohepten-5-äthanol, 103,7 g p-Toluolsulfochlorid und 7,2 g Benzyltriäthylammoniumchlorid in 1440 ml Methylenchlorid gibt man eine Lösung von 664 g Natriumhydroxid in 996 ml Wasser zu und rührt das Gemisch über Nacht bei Raumtemperatur. Das Reaktionsgemisch wird mit 2 l Eiswasser versetzt und mit 3 l Methylenchlorid ausgeschüttelt. Die organischen Auszüge werden mit Wasser neutral gewaschen. Die verbleibenden wäßrigen Auszüge werden anschließend dreimal mit je 2 l Methylenchlorid zurückextrahiert. Die organischen Auszüge werden vereinigt, über Magnesiumsulfat getrocknet und eingedampft. Nach viermaligem Umkristallisieren des festen Rückstandes aus Toluol erhält man reines 2-(4,5,10,11-Tetrahydro-1H-dibenzo[a,d]cyclohepten-5-yl)äthyl-p-toluolsulfonat vom Schmelzpunkt 150—151°.

d) Eine Suspension von 59,8 g 2-(4,5,10,11-Tetrahydro-1H-dibenzo[a,d]cyclohepten-5-yl)äthyl-p-toluolsulfonat in 180 ml Pyrrolidin wird während 21 Stunden bei Raumtemperatur unter Argon gerührt, anschließend auf Eis und 300 ml Wasser gegossen und zweimal mit je 1,2 l destilliertem Äther ausgeschüttelt. Die organischen Auszüge werden zweimal mit je 400 ml Wasser und wenig Eis gewaschen, vereinigt, über Magnesiumsulfat getrocknet und eingedampft. Den Rückstand nimmt man zweimal in je 100 ml trockenem Toluol auf und dampft die erhaltenen Lösungen jeweils zur Trockne ein. Man erhält 1-[2-(4,5,10,11-Tetrahydro-1H-dibenzo[a,d]cyclohepten-5-yl)äthyl]pyrrolidin in Form eines Öles das beim Stehenlassen kristallisiert; Smp. 56—57°. Durch Umkristallisieren aus Pentan erhält man ein Produkt vom Schmelzpunkt 62°.

e) In Analogie zu den Angaben in Beispiel 1j) erhält man 1-[2-(4,5,10,11-Tetrahydro-1H-dibenzo[a,d]cyclohepten-5-yl)äthyl]pyrrolidin-maleat vom Schmelzpunkt 129—130°.

Beispiel 5

a) Zu einer bei 0° unter Argon gerührten Suspension von 35,6 g Lithiumaluminiumhydrid in 1,5 l trockenem Tetrahydrofuran tropft man über einen Zeitraum von 90 Minuten eine Lösung von 237 g 10,11-Dihydro-5H-dibenzo[a,d]cyclohepten-5-essigsäure in 1 l trockenem Tetrahydrofuran zu. Das Reaktionsgemisch wird anschließend während 90 Minuten unter Rückfluß zum Sieden erhitzt, abgekühlt und nacheinander tropfenweise mit 35,6 ml Wasser, 35,6 ml 15proz. Natronlauge und 106,8 ml Wasser versetzt. Die entstandene Suspension wird unter mehrmaligem Nachwaschen mit 2 l Tetrahydrofuran filtriert. Nach Entfernen des Lösungsmittels im Vakuum nimmt man den Rückstand dreimal in je 500 ml Toluol auf, wobei die erhaltenen Lösungen jeweils wieder eingedampft werden. Man erhält reines 10,11-Dihydro-5H-dibenzo[a,d]cyclohepten-5-äthanol in Form eines Öles, das langsam kristallisiert.

b) Das erhaltene Material wird in Analogie zu den Angaben in den Beispielen 4b) bis 4f) in 1-[2-(4,5,10,11-Tetrahydro-1H-dibenzo[a,d]cyclohepten-5-yl)äthyl]pyrrolidin übergeführt.

## Beispiel 6

a) Zu einer unter Argon gerührten, auf 10° gekühlten Lösung von 39 g Natrium in 850 ml trockenem Äthanol gibt man über einen Zeitraum von 60 Minuten eine Lösung von 208,2 g Dibenzosuberon in 600 ml trockenem Äthanol tropfenweise hinzu. Man rührt während 60 Minuten bei einer Temperatur von 10° und versetzt anschließend über einen Zeitraum von 5 Minuten mit 318 g Triäthylphosphonoacetat in 400 ml Äthanol (Horner-Reagens). Man rührte noch 261 Stunden bei Raumtemperatur, wobei man jeweils nach 24, 101, 173 und 197 Stunden mit je 85 ml Horner-Reagens versetzt. Anschließend wird die Reaktionsmischung eingedampft, und der Rückstand auf Eis und 500 ml Wasser gegossen. Nach dreimaligem Ausschütteln mit je 1 l Hexan werden die organischen Auszüge mit 500 ml Wasser gewaschen, vereinigt, über Magnesiumsulfat getrocknet und eingedampft. Das erhaltene Rohprodukt wird aus 250 ml Petroläther (tiefsiedend) umkristallisiert, wobei man kristallines 10,11-Dihydro-5H-dibenzo[a,d]cyclohepten-5-yliden-essigsäureäthylester vom Schmelzpunkt 54° erhält.

b) Eine Lösung von 516,8 g 10,11-Dihydro-5-dibenzo[a,d]cyclohepten-5-yliden-essigsäureäthylester in 1,5 l Äthanol wird unter Rühren über 51,7 g 5-proz. Palladiumkohle während 48 Stunden bei Raumtemperatur und Normaldruck hydriert. Nach Filtrieren und Eindampfen der Reaktionsmischung erhält man 10,11-Dihydro-5H-dibenzo[a,d]cyclohepten-5-essigsäureäthylester in Form eines Öles, das ohne weitere Reinigung in die nächste Stufe eingesetzt wird.

c) 513,2 g 10,11-Dihydro-5H-dibenzo[a,d]cyclohepten-5-essigsäureäthylester werden mit einer Lösung von 130 g Kaliumhydroxid in 900 ml Äthanol und 300 ml Wasser versetzt. Das Gemisch wird während 5,5 Stunden unter Rückfluß zum Sieden erhitzt, auf Eis und 1,5 l Wasser gegossen und einmal mit Äther extrahiert. Die organische Phase wird einmal mit 500 ml Wasser zurückextrahiert. Die wäßrigen Phasen werden vereinigt, mit 300 ml 50-proz. Schwefelsäure sauer gestellt und zweimal mit je 3 l Äther ausgeschüttelt. Die ätherischen Auszüge werden mit 1 l Wasser gewaschen, vereinigt, über Magnesiumsulfat getrocknet und eingedampft. Aus dem erhaltenen Rohprodukt resultiert, nach Umkristallisieren aus Tetrahydrofuran/Hexan, 10,11-Dihydro-5H-dibenzo[a,d]cyclohepten-5-essigsäure vom Schmelzpunkt 163—165°.

Durch Einengen der Mutterlauge im Vakuum erhält man eine zweite Portion Produkt vom Schmelzpunkt 163—165°.

d) Eine unter Argon gerührte und mit Eiswasser gekühlte Lösung von 446,4 g 10,11-Dihydro-5H-dibenzo[a,d]cyclohepten-5-essigsäure in 2,2 l Tetrahydrofuran wird portionsweise mit 87,0 g Natriumborhydrid versetzt. Man rührt das Gemisch während 30 Minuten und tropft anschließend über einen Zeitraum von 90 Minuten bei einer Temperatur von 10° eine Lösung von 290 ml frisch destilliertem Bortrifluoridätherat in 300 ml trockenem Tetrahydrofuran hinzu. Das Reaktionsgemisch wird während 1 Stunde bei 0° und während 16 Stunden bei Raumtemperatur gerührt, auf 0° abgekühlt und über einen Zeitraum von 30 Minuten tropfenweise mit 400 ml Methanol versetzt. Man rührt noch während 15 Minuten bei 0° und 15 Minuten bei Raumtemperatur und dampft die Lösung anschließend im Vakuum ein. Man gießt den Rückstand auf Eis und 1 l Wasser, extrahiert dreimal mit je 2 l Äther, wäscht die organischen Auszüge nacheinander mit 1 l 1,5 N Salzsäure, 1 l 1,5 N Kaliumbicarbonatlösung und mit 1 l Wasser. Die organischen Phasen werden vereinigt, über Magnesiumsulfat getrocknet und eingedampft. Man erhält 10,11-Dihydro-5H-dibenzo[a,d]cyclohepten-5-äthanol in Form eines Öles, das beim Stehenlassen langsam kristallisiert.

e) Das erhaltene Material wird in Analogie zu den Angaben in den Beispielen 4b) bis 4f) in 1-[2-(4,5,10,11-Tetrahydro-1H-dibenzo[a,d]cyclohepten-5-yl)äthyl]pyrrolidin übergeführt.

## Beispiel 7

a) Zu einer in Eiswasser gekühlten, unter Argon gerührten Lösung von 160,36 g 10,11-Dihydro-5H-dibenzo[a,d]cyclohepten-5-essigsäureäthylester in 1 l trockenem Toluol tropft man über einen Zeitraum von 1 Stunde unter Kühlen 1120 ml einer 20-proz. (v/v) Lösung von Diisobutylaluminiumhydrid in Toluol zu. Die Reaktionslösung wird anschließend während 16,5 Stunden bei Raumtemperatur gerührt, vorsichtig unter Kühlen mit 20 ml trockenem Methanol versetzt, während weiterer 30 Minuten gerührt und schließlich vorsichtig unter gutem Kühlen mit 1,5 l 3N Salzsäure angesäuert. Die organische Phase wird abgetrennt und mit 1 l Wasser gewaschen; die saure wäßrige Phase wird noch zweimal mit je 2 l Äther zurückextrahiert. Nach Waschen der ätherischen Auszüge mit 1 l Wasser werden die organischen Phasen vereinigt, über Magnesiumsulfat getrocknet und eingedampft. Der Rückstand wird noch zweimal in je 200 ml trockenem Toluol aufgenommen, wobei die erhaltenen Lösungen jeweils wieder zur Trockne eingedampft werden. Man erhält 10,11-Dihydro-5H-dibenzo[a,d]cyclohepten-5-äthanol in Form eines Öles, das beim Stehenlassen nur langsam kristallisiert.

b) Das erhaltene Material wird in Analogie zu den Angaben in den Beispielen 4b) bis 4f) in 1-[2-(4,5,10,11-Tetrahydro-1H-dibenzo[a,d]cyclohepten-5-yl)äthyl]pyrrolidin übergeführt.

Beispiel 8

In einem 100-ml-Glasgefäß mit abnehmbarem Deckel mit Schliffföffnungen, versehen mit Trockeneiskühler, Thermometer, Platinblechanode und -kathode (je 2,5 × 2,5 cm, Abstand 2 cm) werden 2,0 g 1-[2-(10,11-Dihydro-5H-dibenzo[a,d]cyclohepten-5-yl)-äthyl]pyrrolidin und 2,5 g Lithiumchlorid vorgelegt. Anschließend leitet man während 20 Minuten Stickstoff durch die Apparatur, stellt den Rezipienten in ein Trockeneis/Alkohol-Bad, beschickt den Trockeneiskühler mit Trockeneis und destilliert unter Rühren mit einem Magnetrührer 70 g Methylamin aus der Druckflasche direkt in die Apparatur. Anschließend wird ein Strom von 2 A angelegt, worauf sich eine Spannung von 44 V einstellt und die Kathode sich sofort tiefblau färbt. Die Temperatur wird auf —10° gehalten. Nach dem Durchgang einer Strommenge von 3000 As (entsprechend 230% der theoretisch für den vollen Umsatz benötigten Strommenge) wird der Strom abgestellt und die Reaktionslösung bei 50° und leichtem Vakuum eingedampft. Der Rückstand wird während einiger Minuten im Ultraschallbad in Hexan digeriert. Nach Filtrieren und Eindampfen erhält man rohes 1-[2-(4,5,10,11-Tetrahydro-1H-dibenzo[a,d]cyclohepten-5-yl)-äthyl]pyrrolidin, das in Analogie zu den Angaben in den Beispielen 1j bis 1l) gereinigt wird.

Beispiel A

1-[2-(4,5,10,11-Tetrahydro-1H-dibenzo[a,d]cyclohepten-5-yl)äthyl]pyrrolidin-maleat kann wie folgt als Wirkstoff zur Herstellung von pharmazeutischen Präparaten verwendet werden:

| Kapseln | mg/Kapsel |
|---|---|
| Wirkstoff | 6,98 |
| Maisstärke | 20,00 |
| Milchzucker pulv. | 40,00 |
| Milchzucker krist. | 68,02 |
| Talk | 4,50 |
| Magnesiumstearat | 0,50 |
| Kapselfüllgewicht | 140,00 |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Größe abgefüllt.

**Patentansprüche für die Vertragsstaaten: BE, CH, DE, FR, GB, IT, LI, LU, NL, SE**

1. 1-[2-(4,5,10,11-Tetrahydro-1H-dibenzo[a,d]cyclohepten-5-yl)äthyl]pyrrolidin der Formel I

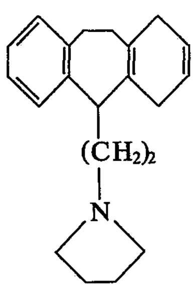

(I)

und pharmazeutisch annehmbare Säureadditionssalze davon.

2. 1-[2-(4,5,10,11-Tetrahydro-1H-dibenzo[a,d]cyclohepten-5-yl)äthyl]pyrrolidinmaleat.

3. 1,4,10,11-Tetrahydro-5H-dibenzo[a,d]cyclohepten.

4. Verbindungen der allgemeinen Formel III

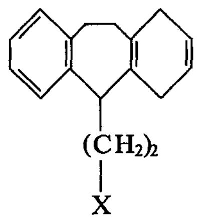

(III)

worin X ein Halogenatom oder einen Sulfonsäurerest bedeutet.

5. Verbindungen gemäß Anspruch 1 oder 2 als pharmazeutische Wirkstoffe.

6. Verbindungen gemäß Anspruch 1 oder 2 als Histamin-$H_1$ antagonistische Wirkstoffe.

7. Verfahren zur Herstellung von Verbindungen gemäß Anspruch 1 oder 2, dadurch gekennzeichnet, daß man

a) 1-[2-(10,11-Dihydro-5H-dibenzo[a,d]cyclohepten-5-yl)-äthyl]pyrrolidin 1,4-reduziert, oder

b) 1,4,10,11-Tetrahydro-5H-dibenzo[a,d]cyclohepten in Gegenwart einer starken Base mit einer Verbindung der allgemeinen Formel II

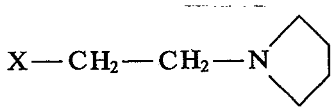

(II)

worin X eine Abgangsgruppe bedeutet,

umsetzt, oder

c) eine Verbindung der allgemeinen Formel III

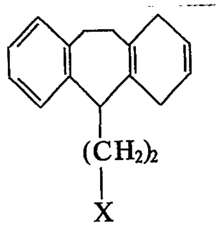

(III)

worin X obige Bedeutung besitzt,

mit Pyrrolidin umsetzt, und

d) das so erhaltene 1-[2-(4,5,10,11-Tetrahydro-1H-dibenzo[a,d]cyclohepten-5-yl)äthyl]pyrrolidin als freie Base oder als pharmazeutisch annehmbares Säureadditionssalz isoliert.

8. Arzneimittel, enthaltend eine Verbindung gemäß Anspruch 1 oder 2.

9. Histamin-$H_1$ antagonistische Mittel, enthaltend eine Verbindung gemäß Anspruch 1 oder 2.

**Patentansprüche für den Vertragsstaat: AT**

1. Verfahren zur Herstellung von 1-[2-(4,5,10,11-Tetrahydro-1H-dibenzo[a,d]cyclohepten-5-yl)äthyl]pyrrolidin der Formel I

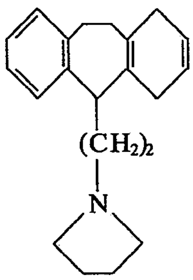

(I)

und pharmazeutisch annehmbare Säureadditionssalze davon, dadurch gekennzeichnet, daß man

a) 1-[2-(10,11-Dihydro-5H-dibenzo[a,d]cyclohepten-5-yl)-äthyl]pyrrolidin 1,4-reduziert, oder
b) 1,4,10,11-Tetrahydro-5H-dibenzo[a,d]cyclohepten in Gegenwart einer starken Base mit einer Verbindung der allgemeinen Formel II

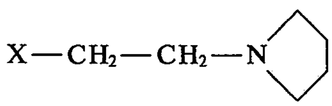

(II)

worin X eine Abgangsgruppe bedeutet,

umsetzt, oder

c) eine Verbindung der allgemeinen Formel III

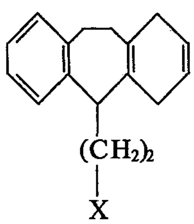

(III)

worin X obige Bedeutung besitzt,

mit Pyrrolidin umsetzt, und

d) das so erhaltene 1-[2-(4,5,10,11-Tetrahydro-1H-dibenzo[a,d]cyclohepten-5-yl)äthyl]pyrrolidin als freie Base oder als pharmazeutisch annehmbares Säureadditionssalz isoliert.

2. Verfahren gemäß Anspruch 1, dadurch gekennzeichnet, daß man 1-[2-(4,5,10,11-Tetrahydro-1H-dibenzo[a,d]cyclohepten-5-yl)äthyl]pyrrolidinmaleat herstellt.

**Claims for the Contracting States: BE, CH, DE, FR, GB, IT, LI, LU, NL, SE**

1. 1-[2-(4,5,10,11-Tetrahydro-1H-dibenzo[a,d]cyclohepten-5-yl)ethyl]pyrrolidine of formula I

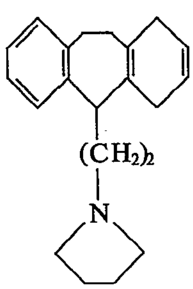

(I)

and pharmaceutically acceptable acid addition salts thereof.

2. 1-[2-(4,5,10,11-Tetrahydro-1H-dibenzo[a,d]dicyclohepten-5-yl)ethyl]pyrrolidine maleate.
3. 1,4,10,11-Tetrahydro-5H-dibenzo[a,d]cycloheptene.
4. Compounds of general formula III

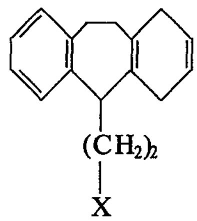

(III)

wherein X signifies a halogen atom or a sulphonic acid residue.

5. Compounds in accordance with claim 1 or 2 as pharmaceutically active substances.
6. Compounds in accordance with claim 1 or 2 as active substances which antagonize histamine-$H_1$.
7. A process for the manufacture of compounds in accordance with claim 1 or 2, characterized by

a) 1,4-reducing 1-[2-(10,11-dihydro-5H-dibenzo[a,d]cyclohepten-5-yl)ethyl]pyrrolidine, or

b) reacting 1,4,10,11-tetrahydro-5H-dibenzo[a,d]cycloheptene in the presence of a strong base with a compound of general formula II

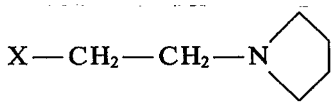

(II)

wherein X signifies a leaving group,

or

c) reacting a compound of general formula III

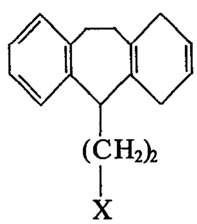

(III)

wherein X has the above significance,

with pyrrolidine, and

d) isolating the thus-obtained 1-[2-(4,5,10,11-tetrahydro-1H-dibenzo[a,d]cyclohepten-5-yl)ethyl]pyrrolidine as the free base or as a pharmaceutically acceptable acid addition salt.

8. A medicament containing a compound in accordance with claim 1 or 2.
9. A medicament which antagonizes histamine-$H_1$, containing a compound in accordance with claim 1 or 2.

**Claims for the Contracting State: AT**

1. A process for the manufacture of 1-[2-(4,5,10,11-tetrahydro-1H-dibenzo[a,d]cyclohepten-5-yl)ethyl]pyrrolidine of formula I

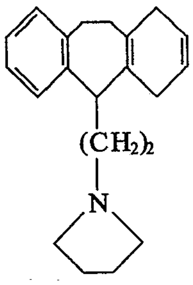

(I)

and pharmaceutically acceptable acid addition salts thereof, characterized by

a) 1,4-reducing 1-[2-(10,11-dihydro-5H-dibenzo[a,d]cyclohepten-5-yl)-ethyl]pyrrolidine, or
b) reacting 1,4,10,11-tetrahydro-5H-dibenzo[a,d]cycloheptene in the presence of a strong base with a compound of general formula II

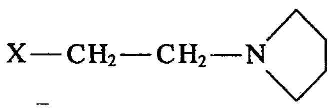

(II)

wherein X signifies a leaving group.

c) reacting a compound of general formula III

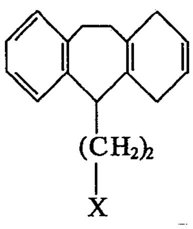

(III)

wherein X has the above significance,

with pyrrolidine, and

d) isolating the thus-obtained 1-[2-(4,5,10,11-tetrahydro-1H-dibenzo[a,d]cyclohepten-5-yl)ethyl]pyrrolidine as the free base or as a pharmaceutically acceptable acid addition salt.

2. A process in accordance with claim 1, characterized in that 1-[2-(4,5,10,11-tetrahydro-1H-dibenzo[a,d]cyclohepten-5-yl)ethyl]pyrrolidine maleate is manufactured.

**Revendications pour les Etats contractants: BE, CH, DE, FR, GB, IT, LI, LU, NL, SE**

1. 1-[2-(4,5,10,11-Tétrahydro-1H-dibenzo[a,d]cycloheptén-5-yl)éthyl]pyrrolidine de formule I

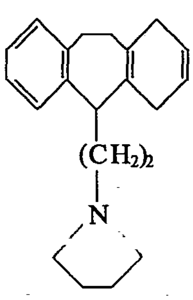

(I)

et ses sels d'addition d'acides pharmaceutiquement acceptables.

2. 1-[2-(4,5,10,11-Tétrahydro-1H-dibenzo[a,d]cycloheptén-5-yl)éthyl]pyrrolidinemaléate.
3. 1,4,10,11-Tétrahydro-5H-dibenzo[a,d]cycloheptène.
4. Composés de formule générale III

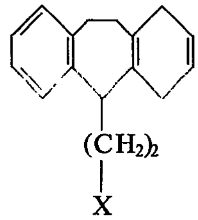

(III)

où X représente un atome d'halogène ou un radial acide sulfonique.

5. Composés selon les revendications 1 et 2 comme substances actives pharmaceutiques.
6. Composés selon les revendications 1 et 2 comme substances actives antagonistes de l'histamine-H1.
7. Procédé de préparation de composés selon la revendication 1 ou 2, caractérisé en ce que

a) on réduit en 1,4 la 1-[2-(10,11-dihydro-5H-dibenzo[a,d]cycloheptén-5-yl)éthyl]pyrrolidine, ou

b) on fait réagir le 1,4,10,11-Tétrahydro-5H-dibenzo[a,d]cycloheptène en présence d'une base forte avec un composé de formule générale II

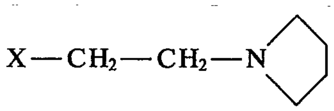

(II)

où X représente un groupe sortant, ou

c) on fait réagir un composé de formule générale III

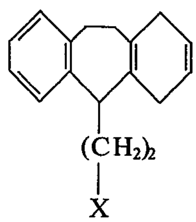

(III)

où X a la signification donnée ci-dessus,

avec de la pyrrolidine, et

d) on isole la 1-[2-(4,5,10,11-tétrahydro-1H-dibenzo[a,d]cycloheptén-5-yl)éthyl]pyrrolidine ainsi obtenue sous forme de base libre ou sous forme de sel d'addition d'acide pharmaceutiquement acceptable.

8. Médicament contenant un composé selon la revendication 1 ou 2.
9. Agent antagoniste de l'histamine-H1 contenant un composé selon la revendication 1 ou 2.

**Revendications pour l'Etat contractant: AT**

1. Procédé de préparation de 1-[2-(4,5,10,11-tétrahydro-1H-dibenzo[a,d]cycloheptén-5-yl)éthyl]pyrrolidine de formule I

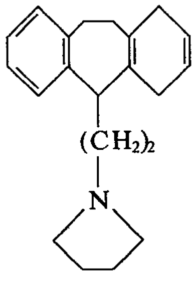

(I)

et de ses sels d'addition d'acides pharmaceutiquement acceptables, caractérisé en ce que

a) on réduit en 1,4 la 1-[2-(10,11-dihydro-5H-dibenzo[a,d]cycloheptén-5-yl)éthyl]pyrrolidine, ou

b) on fait réagir le 1,4,10,11-tétrahydro-5H-dibenzo[a,d]cycloheptène en présence d'une base forte avec un composé de formule générale II

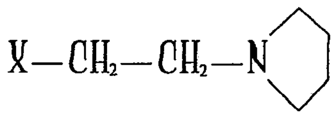

(II)

où X représente un groupe sortant, ou

c) on fait réagir un composé de formule générale III

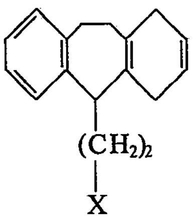

(III)

où X a la signification donnée ci-dessus,

avec de la pyrrolidine, et

d) on isole la 1-[2-(4,5,10,11-tétrahydro-1H-dibenzo[a,d]cycloheptén-5-yl)éthyl]pyrrolidine ainsi obtenue sous forme de base libre ou sous forme de sel d'addition d'acide pharmaceutiquement acceptable.

2. Procédé selon la revendication 1, caractérisé en ce qu'on prépare le 1-[2-(4,5,10,11-tétrahydro-1H-dibenzo[a,d]cycloheptén-5-yl)éthyl]pyrrolidine maléate.